# EUROPEAN PATENT SPECIFICATION

(11) **EP 2 085 039 B1**
(45) Date of publication and mention of the grant of the patent: **19.03.2014**
(21) Application number: 09001310.3
(22) Date of filing: 30.01.2009
(51) Int. Cl.: A61B 17/68, A61B 17/86, A61B 17/00

(54) **Bone fixation device**
Knochenfixiervorrichtung
Dispositif de fixation d'os

(30) Priority: 04.02.2008 US 25198
(43) Date of publication of application: 05.08.2009
(73) Proprietor: Chiu, Mark Hsien Nien, Surrey BC V3S 7Y6 (CA); Schemitsch, Emil, Thornhill ON L3T 1N1 (CA)
(72) Inventor: Chiu, Mark Hsien Nien, Surrey British Columbia V3S 7Y6 (CA)
(74) Representative: Coyle, Philip Aidan

(56) References cited:
- EP-A- 1 273 269
- WO-A-2006/105935
- WO-A-2008/057404
- DE-U1- 20 009 626

## Description

### FIELD OF THE INVENTION

The invention relates to an apparatus for flexibility securing a tibia to a fibula at an ankle syndesmosis.

### BACKGROUND OF THE INVENTION

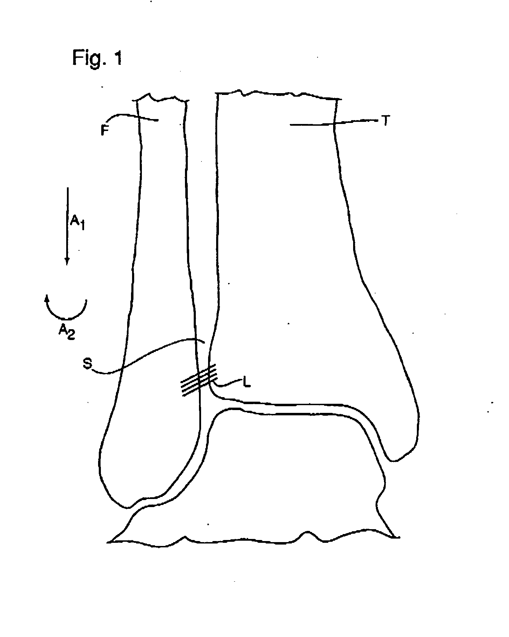

The ankle syndesmosis (S) is the joint between the distal tibia (T) and the distal fibula (F), where the tibia and fibula are held together by connective ligaments (L), as shown in Figure 1. At the ankle syndesmosis, the tibia and the fibula are supported and held together by interosseous membrane and three main ligaments: the anterior inferior tibiofibular ligament, the posterior inferior tibiofibular ligament, and the transverse ligament. During normal gait, the fibula moves with respect to the tibia. For example, referring to Figure 1, body weight and muscle contraction during walking actively pulls the fibula (F) downward relative to the tibia (T), in a direction indicated by arrow A₁. Additionally, during the phase of walking at which ankle dorsiflexion occurs, the fibula rotates in a plane transverse to the direction of arrow A₁ with respect to the tibia, in a direction indicated by arrow A₂ , which is external rotation. The amount that the fibula moves is variable, however some studies indicate that the fibula may move in direction A₁ by about 1 - 2 mm and in direction A₂ by about 2 degrees.

A common type of ankle injury is injury to the ankle syndesmosis. This type of injury is sometimes called a high ankle sprain. In an ankle syndesmosis injury, at least one of the ligaments connecting the bottom ends of the tibia and fibula bones is tom. Disruption is caused by external rotation forces on the foot and ankle. Progressive disruption of these ligaments results in increased diastasis and instability. Mild cases of ankle syndesmosis injury without instability and widening of the syndesmotic space are often treated non-surgically. However, more serious cases may require surgery. Typical surgery to repair the ankle syndesmosis involves placement of one or more stainless steel or bioabsorbable screws through the fibula (F), across the syndesmotic space (S), and into the tibia (T).

EP 1 273 269 A2 entitled "Implantable screw for stabilising an articulated joint or a bone fracture" is directed to an implantable screw includes an elongated body with a proximal head portion (1) and a distal threaded portion (2) having a thread. The head portion and threaded portion are interconnected by a flexible shaft. However, EP 1 273 269 A2 does not disclose a unitary body with a connector made of a solid nitinol body. EP 1 273 269 A2 instead discloses a multi-piece connector which is designed to supply a central portion with flexibility without the transfer/transmission of compressive forces through the flexible shaft.

### SUMMARY OF THE INVENTION

In accordance with a broad aspect, a bone fixation device is provided. The bone fixation device comprises a longitudinally extending nitinol body having a longitudinally extending axis. The bone fixation device further comprises a first portion comprising an outer surface having at least one bone engagement member securable to the tibia, and a second portion comprising an outer surface having at least one bone engagement member securable to the fibula. A central portion extends between the first portion and the second portion and positionable between the tibia and the fibula
wherein the central portion is configured to deflect from the longitudinal axis to allow the fibula to move relative to the tibia in a direction transverse to the longitudinal axis by a distance comparable to a natural movement of the tibia relative to the fibula at the syndesmotic joint; and
wherein the first portion defines a first outer diameter, the second portion defines a second outer diameter, and the central portion defines a third outer diameter less than the first and second outer diameters.

Embodiments in accordance with this broad aspect may be advantageous because the central portion may be substantially flexible, and may therefore bend, flex, or deflect to accommodate the movements of the joint Accordingly, the connector may permit more natural range of movement for the joint than current surgical methods, and may promote healing of the joint. Additionally, the connector may have a substantially high fatigue resistance, and may therefore be used in the body for an extended period of time without breaking. Furthermore, the connector may prevent bone shielding which leads to osteopenia. Additionally, the connector may be substantially corrosion resistant.

In some embodiments, the nitinol comprises from 50% to 60% nickel, and from 40% to 50 % titanium by weight.

In some embodiments, the central portion has a length of from 5 to 20 mm. In some further embodiments, the tibia and has a length of from 20 to 50 mm and the fibula has a length of from 5 to 20 mm.

In some embodiments, the central portion has an absence of screw threads.

In some embodiments, the bone fixation device has a cycle fatigue of at least 400,000 cycles.

In some embodiments, the bone engagement members comprise screw threads. In some further embodiments, the bone fixation comprises a screw, preferably wherein the central portion has an absence of screw threads.

In some embodiments, the bone fixation device comprises a head adjacent the second portion comprising a slot for receiving a tool. In further embodiments, the first end comprises an additional slot for receiving a tool.

In some embodiments, the bone fixation device extends linearly.

In another broad aspect, a bone fixation device is provided that comprises a longitudinally extending body comprising a first portion comprising an outer surface having at least one bone engagement member securable to a first bone; a second portion comprising an outer surface having at least one bone engagement member securable to a second bone; and, a central portion extending between the first portion and the second portion, the central portion being fabricated from a material having a modulus of elasticity of between 20GPa and 80GPa.

In some embodiments, the bone fixation device has a rigidity that is sufficient to secure the first bone and the second bone in a normal anatomical position during normal movement of a portion of a body containing the first bone and the second bone.

In some embodiments, the first bone is a tibia, the second bone is a fibula, and the normal movement is a swing phase of walking.

In some embodiments, the bone fixation device is a screw, and at least one of the bone engagement members comprises a screw thread.

In some embodiments, a diameter of the central portion is at least 2 mm.

In another broad aspect (not claimed), the use of a connector for repairing a syndesmotic joint of an ankle is provided. The connector comprises a unitary nitinol body and having, spaced sequentially, a first portion securable to a tibia of a patient proximate the syndesmotic joint; a central portion, at least a portion of which is positioned in a syndesmotic space of the syndesmotic joint; and, a second portion securable to a fibula of the patient, the portions defining a longitudinally extending axis.

In some embodiments, each of the first portion and the second portion comprise screw threads on an outer surface thereof.

In some embodiments, the central portion has a flexibility to secure the tibia and fibula in a spaced apart relationship on opposed sides of the syndesmotic space such that when normal physiologic forces are applied to the syndesmotic joint, the tibia moves with respect to the fibula by at least 40% of an uninjured syndesmotic joint.

In some embodiments, the central portion has a modulus of elasticity of 20 - 80 GPa.

In some embodiments, the central portion has a modulus of elasticity of 30 - 40 GPa.

In some embodiments, the central portion has an external surface having an absence of screw threads.

In some embodiments, the central portion is configured to deflect from the longitudinal axis when a force is applied transverse to the longitudinal axis while the first and second portions remain secured in position and to re-align with the longitudinal axis when the force is removed, and to undergo at least 400,000 cycles of deflection and re-alignment without breaking.

In some embodiments, the central portion has a length such that a portion is embeddable within at least one of the tibia and the fibula.

In some embodiments, the central portion has a length such that a portion is embeddable within the fibula and another portion of the central portion is embedded within the tibia.

### BRIEF DESCRIPTION OF THE DRAWINGS

These and other advantages of the present invention will be more fully and particularly understood in connection with the following description of the preferred embodiments of the invention in which:

Figure 1 is a front plan view of a syndesmotic joint of a human;

Figure 2 is a front plan view of an embodiment of a bone fixation device of the present invention;

Figure 3 is a front plan view of an alternate embodiment of a bone fixation device of the present invention;

Figure 4A is a front plan view of an embodiment of a bone fixation device of the present invention positioned in a syndesmotic joint of a human, showing the tibia and fibula in a rest position;

Figure 4B is a front plan view of an embodiment of a bone fixation device of the present invention positioned in a syndesmotic joint of a human, showing the fibula vertically displaced from the tibia;

Figure 5A is a top plan view of an embodiment of a bone fixation device of the present invention positioned in a syndesmotic joint of a human, showing the tibia and fibula in a rest position; and

Figure 5B is a top plan view of an embodiment of a bone fixation device of the present invention positioned in a syndesmotic joint of a human, showing the fibula rotationally displaced from the tibia;

### DETAILED DESCRIPTION OF THE INVENTION

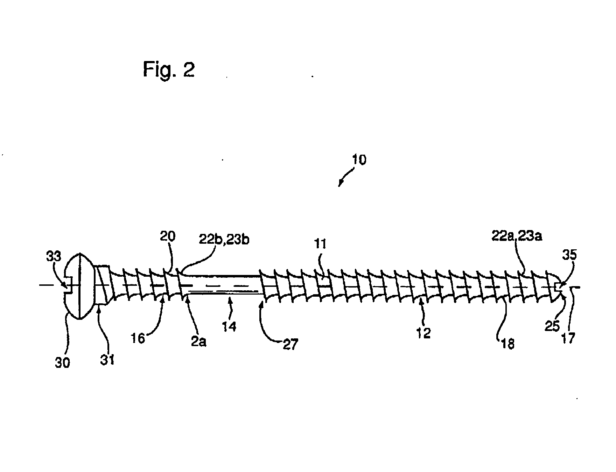

Referring to Figure 2, an embodiment of a bone fixation device 10 of the present invention is shown. Bone fixation device 10 is a connector comprising a unitary body 11, and is configured to repair injuries, such as ankle syndesmosis injuries, by flexibly connecting two bones, such as the tibia and the fibula. Bone fixation device 10 has a longitudinally extending axis 17, and comprises a first portion 12, a central portion 14, and a second portion 16. Central portion 14 extends along axis 17 between first portion 12 and second portion 16. Bone fixation device 10 is configured such that in use, first portion 12 is secured to a first bone, second portion 16 is secured to a second bone, and at least a portion of central portion 14 extends between the bones.

In order to secure each of first portion 12 and second portion 16 to first and second bones, each of first portion 12 and second portion 16 preferably have an outer surface 18, 20 comprising at least one bone engagement member 22a, 22b. By providing each of first 12 and second 16 portions with a bone engagement member, rather than only first portion 12, over compression or over tightening of the bones may be minimized or prevented.

In the embodiment shown in Figure 2, bone engagement members 22a, 22b comprise screw threads 23a, 23b, which extend outwardly from outer surfaces 18, 20. Accordingly, bone fixation device 10 is secured to first and second bones, by screwing first portion 12 through a first bone and then into second bone, and screwing second portion 16 into the first bone, such that central portion 14 is positioned at least partially between first bone and second bone.

Screw threads 23a, 23b, may be of a variety of configurations. In the embodiment shown, screw threads 23a extend along first portion 12 from a position adjacent a first end 25 of first portion 12 to a position adjacent a second end 27 of first portion 12. Screw threads 23b extend along second portion 16 from a position adjacent a first end 29 of second portion 16 to a position adjacent a second end 31 of second portion 12. In alternate embodiments, screw threads 23a, 23b may extend along only a portion of each of first portion 12 and second portion 16. In the preferred embodiment, the screw threads are configured to have a diameter and thread pitch compatible with existing orthopedic systems, such that existing tools may be used. Accordingly, in some embodiments, the pitch of screw threads 23a, 23b is between 0.5 mm and 2.5 mm. In the preferred embodiment, screw threads 23a, 23b have a pitch of about 1.25mm. Furthermore, the screw threads are preferably buttress shaped.

In alternate embodiments, bone engagement members 22a, 22b may be another member other than a screw thread, such as one or more hooks, barbs, or pins. For example, as shown in Figure 2, each of first portion 12 and second portion 16 may comprise one or more barbs 28 extending outwardly from outer surface 18 of first portion 12 and outer surface 20 of second portion 16, and being angled in a direction towards optional head 30. Barbs 28 may be configured such that when bone fixation device is inserted into first and second bones, for example using a hammer, barbs 28 provide minimal resistance to oppose the insertion; however, if force was applied in a direction indicated by arrow A to bone fixation device 10 to remove it from the first and second bones, barbs 28 would engage the bones and resist the motion. In yet alternate embodiments, connector 10 may be a dowel, and bone engagement members may comprise the outer surfaces 18, 20 of first 12 and second 16 portions of connector 10, which may be sized to frictionally engage the bones. Preferably, as exemplified, bone fixation device 10 is a screw.

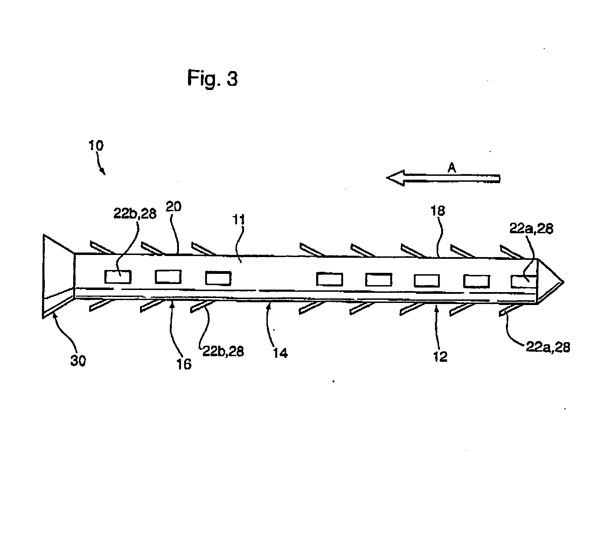

In any of the above-described embodiments, bone fixation device 10 may further comprise a head 30 adjacent second portion 16. In some embodiments, head 30 may comprise a slot 33 for receiving a screwdriver or a drill bit. Alternatively, head 30 may be substantially flat, for striking with a hammer (see for example Figure 3).

In any of the embodiments, first end 25 of first portion 12 may comprise a slot 35 for receiving a tool such as a screwdriver or a drill bit. Such a slot may be useful if connector 10 breaks while in use in the body. In such cases, connector 10 may be removed from the body by unscrewing second portion 16 from the fibula and by unscrewing first portion 12 longitudinally through and out of the tibia

As previously mentioned, central portion 14 extends between first portion 12 and second portion 16. Central portion 14 is configured to deflect from the longitudinal axis to allow the second bone to move relative to the first bone in a direction transverse to the longitudinal axis by a distance that is preferably comparable to a natural movement of the first bone relative to the second bone. Central portion 14 may be of a variety of shapes, and is preferably cylindrical. In order to provide a central portion 14 that has the requisite flexibility, central portion 14 may have a reduced diameter, be made of a more flexible material, and/or be constructed to have a reduced number of, and preferably an absence of, elements that increase the structural rigidity of central portion 14, such as by having an absence of screw threads.

In the embodiment exemplified in Figures 2 and 3, central portion 14 is provided with an absence of screw threads, barbs, or other bone engagement members. That is, the outer surface of central portion 14 is substantially smooth. The provision of screw threads on central portion 14 provides elements that increase the rigidity of the central portion. In alternate embodiments, central portion may be provided with a textured outer surface. For example, the outer surface of central portion 14 may be provided with screw threads.

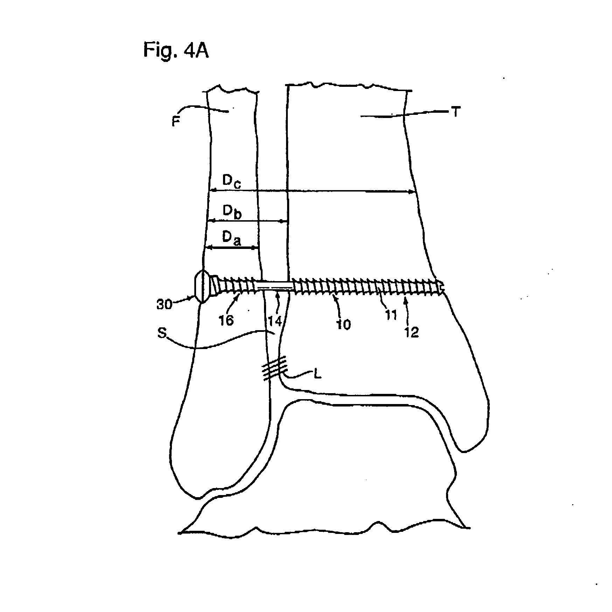

Bone fixation device 10 may be sized depending on the intended use of bone fixation device 10. In some embodiments, as shown in Figure 4A, bone fixation device 10 is preferably used to repair a syndesmotic joint. It will be appreciated that the dimensions of bone fixation device 10 may vary depending if bone fixation device 10 is to be used on an adult or a child. In some such embodiments, it may be desired for the length of central portion 14 to be between about 5 mm in length and 20 mm in length and preferably between about 5mm and about 10 mm in length, in order to span the syndesmotic space (S). Further, it may be desired for central portion 14 to be substantially cylindrical, and to be between about 2 mm and about 3mm in diameter, in order to be easily positioned in the syndesmotic space (S).

Preferably, first portion 12 and second portion 16 may be sized such that they may be secured to the tibia (T) and the fibula (F), respectively. Accordingly, first portion 12 may have a length of between about 20 mm and about 50 mm, and preferably between about 25mm and about 35mm, in order to span the diameter of (i.e. extend through) the tibia (T). Second portion 16 may have a length of about 5 mm to about 20 mm, preferably between about 8 mm and about13 mm, in order to span the diameter of (i.e. extend through) the fibula (F). First portion 12 and second portion 16 may have an outer diameter of between 2 mm and about 3 mm (not including bone engagement members 22), and with bone engagement members 2 may have an outer diameter of between about 3 mm and about 5 mm.

It will be appreciated that bone engagement members 22a, 22b may be provided on only part of first and second portions 12, 16. Further, it will be appreciated that part of central portion 14 may be provided with bone engagement members 22a, 22b. For example, in any embodiment, once inserted into the bones, end 29 may be positioned in the syndesmotic space. Alternately, or in addition, end 27 may be positioned in the syndesmotic space. Since at least part of central portion 14 has, e.g., no screw threads but the same diameter as portion 12, 16 (exclusive of the screw threads), the flexibility of central portion 14 is increased.

Although in the embodiments shown, central portion 14 has substantially the same outer diameter as first and second portions 12, 16 excluding the screw threads on first and second portions (i.e. in the direction transverse to axis 17 between the bases or troughs of the screw threads), in alternate embodiments, central portion 14 may have a diameter that is less than that of first and second portions. Providing central portion 14 with a reduced diameter may serve to increase the flexibility of central portion 14.

As previously mentioned, bone fixation device 10 is configured to flexibly connect a first bone, such as a tibia, and a second bone, such as a fibula, such that if the bones move relative to each other, each of first portion 12 and second portion 16 will remain secured to the bones, and central portion 14 will flex, bend, or deflect to accommodate the movement. More specifically, bone fixation device 10 is configured such that if bone fixation device 10 extends along axis 17, and the first bone moves relative to the second bone, for example in a direction transverse to axis 17, central portion 14 will flex such that bone fixation device deflects from axis 17. Further, bone fixation device 10 may be configured such that if the first bone returns to its original position, central portion 14 will realign with axis 17.

In order to provide central portion 14 with the ability to repeatedly flex, bend, or deflect, at least central portion 14, and preferably all of body 11 of bone fixation device 10 is fabricated from a superelastic or shape memory material such as nitinol (nickel titanium alloy), which has a low modulus of elasticity (preferably from about 20 GPa to about 80 GPa), and a high fatigue resistance (preferably at least 400,000 cycles without failure) Preferably all of body 11 is made of nitinol, which allows connector 10 to flex, bend, or deflect to provide a relatively natural range of movement to joints.

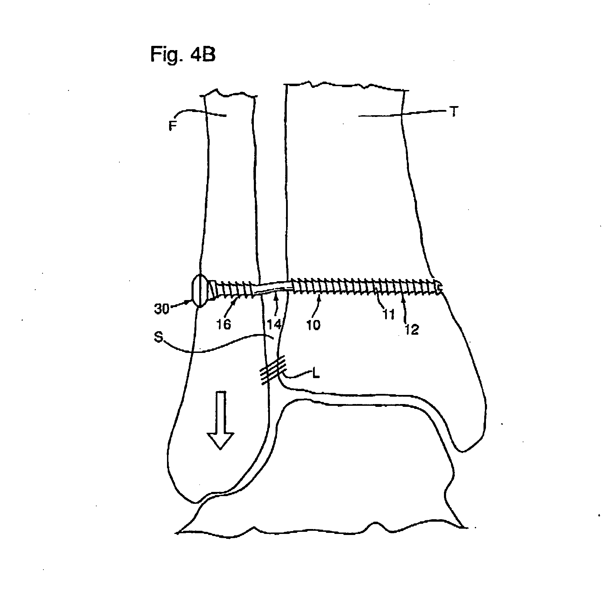

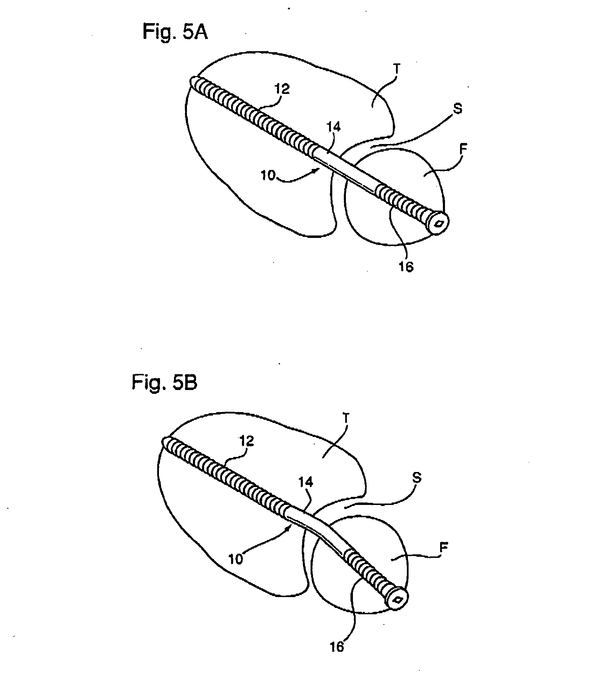

For example, in some embodiments, connector 10 is used to connect the tibia and the fibula to repair the syndesmotic joint of an ankle. As previously mentioned, during normal walking in a human patient, the fibula moves downward (vertically) with respect to the tibia, and externally rotates with respect to the fibula. As shown in Figure 4A and 5A, during the swing phase of walking where the foot is in mid-air, and minimal forces are applied to the joint and the joint is at rest, central portion 14 of connector 10 may be in a substantially straight or linear configuration. During the stance or pushing off phase of walking, when forces are applied to the joint and the fibula moves downward (vertically) and externally rotates with respect to the tibia, central portion 14 may deflect both downwardly, as shown in Figure 4B, and rotationally, as shown in Figure 5B, in order to accommodate the movement and allow the joint to approximate to its natural range of movement. When the swing phase of walking is again resumed, and minimal forces are applied to the joint, the central portion may again return to a straight configuration. The amount of movement which connector 10 allows will depend on the particular configuration of and material used to make connector 10, as well as the nature of the joint being repaired. That is, factors such as scar formation, prolonged immobilization, or severe associated leg injuries may reduce syndesmotic motion. However, in some embodiments, when normal physiologic forces are applied to the joint, connector 10 may allow the tibia to move with respect to the fibula by a distance that is at least 40%, and in some cases 100%, of the distance allowed in the uninjured syndesmotic joint, and by an angle that is at least 40%, and in some cases 100% of the angle allowed in the uninjured syndesmotic joint. In some particular embodiments, connector 10 may allow the fibula to rotate by between about 1 degree and about 4 degrees, and to move downwardly by between about 0.5 mm and 3 mm, and more specifically between about 1 mm and about 2 mm during walking..

In addition to allowing connector 10 to flex, bend, or deflect, the use of nitinol allows connector 10 to be used in the body for an extended period of time, without breaking or otherwise failing. For example, some embodiments of connector 10 may be able to undergo up to 400,000 or more cycles, and preferably more than 1,000,000 cycles, of deflection and re-alignment, without failing. Additionally, due to the low modulus of elasticity of nitinol, the use of connector 10 may reduce bone shielding which leads to osteopenia. Furthermore, the use of nitinol may minimize or prevent corrosion of connector 10 in the body.

The particular alloy of nitinol used for connector 10 may vary depending on the particular application. In some embodiments, bone fixation device is fabricated from a nitinol comprising between 50% to 60% nickel, and from 40% to 50 % titanium by weight. It will be appreciated that any formulation of nitinol known now or in the future maybe used. For example, in some embodiments, the nitinol may comprise additional components other than nickel and titanium, such as one or more standard or known additives. In the preferred embodiment, bone fixation device is fabricated from a nitinol comprising about 45 wt% nickel and about 55 wt% titanium. The nitinol is preferably in the martensitic form at body temperature (37°C), but may be in an austenitic form. The transformation temperature may be above or below 37°C, and is preferably approximately 60°C. In some embodiments, the nitinol may be configured to have a modulus of elasticity of between 20GPa and 80GPa. In the preferred embodiment, the nitinol has a modulus of elasticity of between about 30GPa and 40GPa. Furthermore, in some embodiments, surface processing treatments such as mirror polishing or oxide coating may be applied to connector 10 to further reduce corrosive forces.

As previously mentioned, the entirety of connector 10 may be made from nitinol, or only central portion 14 may be made from nitinol. For example, first and second portions may be made from stainless steel, and may be affixed to central portion 14, for example by welding.

Bone fixation device 10 may have a variety of uses in the body. In some particular embodiments, bone fixation device 10 may be used to repair damaged joints, such as joints that have been sprained. In one particular embodiment, as exemplified herein bone fixation device 10 may be used to repair an ankle syndesmosis, as will presently be described. Other potential uses may include repair of the coracoacromial joint, acromioclavicular joint, and symphysis pubis joints which involve ligaments which allow normal motion between attached bones. It will be appreciated that in any such uses, bone fixation device 10 has a rigidity that is sufficient to secure the first bone and the second bone in a normal anatomical position during normal movement of a portion of a body containing the first bone and the second bone. For example, if bone fixation device 10 is used to repair an ankle syndesmosis, then the bone fixation device has sufficient rigidity to secure the tibia and fibula in position during normal movement of the leg (e.g., the swing phase of walking). The required rigidity will vary depending upon the bones that are to be secured together.

Prior to positioning bone fixation device 10 in the ankle syndesmosis, a variety of optional steps may be performed. In a first step, the ankle syndesmosis may be positioned into its normal configuration by pressing the tibia and fibula together at or just above the level of the ankle. A drill may then be used to create a hole through the fibula and into the tibia. The distance that the hole extends into the tibia may vary depending various factors, for example the type and severity of injury. However, in the preferred embodiment, the hole is drilled through the entirety of the tibia, from the medial tibial cortex to the lateral tibial cortex. The hole may be created through the widest part of the fibula, approximately 2 - 4 cm above the ankle joint line. In some embodiments, a 2.5mm drill bit may be used to create the hole. The hole may be created in an anteromedial direction towards the tibia, and parallel to the joint line. A depth gauge may then be used to measure the distance D_{A} from the lateral fibular cortex to the medial fibular cortex, the distance D_{B} from the lateral fibular cortex to the medial tibial cortex, and the distance D_{C} from the lateral fibular cortex to the lateral tibial cortex (shown in Figure 4A). The distance across the syndesmotic space D_{S} may be approximated by the difference between distance D_{B} and distance D_{A}. An appropriately sized bone fixation device 10 may then be selected based on these measurements. For example, if distance D_{A} is 10 mm, distance D_{B} is 20 mm, and distance D_{C} is 50 mm, a bone fixation device 10 that has a first portion 12 length of 10 mm, a second portion 16 length of 30 mm and a central portion 14 length of 10 mm may be selected.

When the joint has been prepared, bone fixation device 10 may be used to repair the joint by securing first portion 12 to the tibia, securing second portion 16 to the fibula, and positioning at least a portion of central portion 14 in the syndesmotic space. That is, in some embodiments, first portion 12 may be passed through the fibula and into the tibia, central portion 14 may be passed through the fibula and into the syndesmotic space, and second portion 16 may be passed into the fibula. In embodiments wherein bone engagement members 18 comprise screw threads, bone fixation device 10 may be positioned by screwing first portion 12 through the fibula and into the tibia, and screwing second portion 16 into the fibula. In alternate embodiments, bone fixation device 10 may be positioned in another manner, for example by hammering.

Bone fixation device is positioned such that at least a portion of central portion 14 is in the syndesmotic space. In the preferred embodiment, central portion 14 is centered within the syndesmotic space, with a portion extending into and embedded in the tibia, and/or a portion extending into and embedded in the fibula.

In the preferred embodiment, bone fixation device may be positioned such that first portion 12 extends across the entire diameter of the tibia, and end 25 is positioned adjacent the lateral edge of the tibia. Such an embodiment may be advantageous because if bone fixation device 10 breaks while in the body, first portion 12 may be accessed from the medial side of the ankle to remove first portion 12.

After bone fixation device 10 is positioned, and an appropriate healing time has passed, the patient may begin to walk. Due to the ability of central portion 14 to deflect or bend, the patient may be able to walk with greater comfort, and with a more natural gait. Further, the bone fixation device may be able to withstand numerous cycles of walking, without failing. Additionally, due to the low modulus of elasticity of central portion 14, bone shielding which leads to osteopenia may be prevented.

It will be appreciated that certain features of the invention, which are, for clarity, described in the context of separate embodiments or separate aspects, may also be provided in combination in a single embodiment. Conversely, various features of the invention, which are, for brevity, described in the context of a single embodiment or aspect, may also be provided separately or in any suitable subcombination.

Although the invention has been described in conjunction with specific embodiments thereof, if is evident that many alternatives, modifications and variations will be apparent to those skilled in the art. Accordingly, it is intended to embrace all such alternatives, modifications and variations that fall within broad scope of the appended claims. In addition, citation or identification of any reference in this application shall not be construed as an admission that such reference is available as prior art to the present invention.

## Claims

1. A bone fixation device (10) for flexibly securing a tibia to a fibula at an ankle syndesmosis comprising a unitary longitudinally extending nitinol body having a longitudinally extending axis (17) and comprising:
a first portion (12) comprising an outer surface having at least one bone engagement member (22a/22b) securable to the tibia;
a. a second portion (16) comprising an outer surface having at least one bone engagement
member securable to the fibula; and,
b. a central portion (14) extending between the first portion and the second portion and positionable between the tibia and the fibula
wherein the central portion (14) is configured to deflect from the longitudinal axis (17) to allow the fibula to move relative to the tibia in a direction transverse to the longitudinal axis by a distance comparable to a natural movement of the tibia relative to the fibula at the syndesmotic joint; and
wherein the first portion (12) defines a first outer diameter, the second portion defines a second outer diameter, and the central portion (14) defines a third outer diameter less than the first and second outer diameters.

2. The device of claim 1, wherein the nitinol comprises from 50% to 60% nickel, and from 40% to 50% titanium by weight.

3. The device of any of claims 1 and 2, wherein the central portion (14) has a length of from 5 to 20 mm.

4. The device of any of claims 1-3, wherein the first portion (12) has a length of from 20 to 50 mm and the second portion (16) has a length of from 5 to 20 mm.

5. The device of any claims 1-3, wherein the central portion (14) has an absence of screw threads.

6. The device of any of claims 1-5, wherein the bone fixation device (10) has a cycle fatigue of at least 400,000 cycles.

7. The device of any of claims 1-6, wherein the bone engagement members (22a/22b) comprise screw threads (23a/23b).

8. The device of any of claims 1-7, wherein the bone fixation device comprises a screw.

9. The device of any of claims 1-8, further comprising a head adjacent the second portion (16) comprising a slot (35) for receiving a tool.

10. The device of any of claims 1-9, wherein the first portion (12) comprises an additional slot for receiving a tool.

11. The device of any of claims 1-10, wherein the bone fixation (10) device extends linearly.

12. The device of claim 5, wherein the distance is between about 0.5 mm and about 3 mm.

13. The device of any of claims 1-12, wherein the central portion has a modulus of elasticity of 20 - 80 GPa.

## Patentansprüche

1. Knochenfixierungsvorrichtung (10) zum flexiblen Befestigen eines Schienbeins an einem Wadenbein an einer Fußgelenkssyndesmose, umfassend einen unitären, sich in Längsrichtung erstreckenden Nitinolkörper, der eine sich in Längsrichtung erstreckende Achse (17) aufweist und Folgendes umfasst: einen ersten Abschnitt (12), umfassend eine äußere Oberfläche mit mindestens einem Knocheneingriffselement (22a/22b), das an dem Schienbein befestigt werden kann;
a. einen zweiten Abschnitt (16), umfassend eine äußere Oberfläche mit mindestens einem Knocheneingriffselement, das an dem Wadenbein befestigt werden kann; und
b. einen mittleren Abschnitt (14), der sich zwischen dem ersten Abschnitt und dem zweiten Abschnitt erstreckt und zwischen dem Schienbein und dem Wadenbein positioniert werden kann;
wobei der mittlere Abschnitt (14) dazu ausgebildet ist, von der Längsachse (17) ausgelenkt zu werden, um zuzulassen, dass sich das Wadenbein relativ zu dem Schienbein in einer Richtung quer zur Längsachse um eine Strecke bewegt, die mit einer natürlichen Bewegung des Schienbeins relativ zum Wadenbein am Syndesmosegelenk vergleichbar ist; und
wobei der erste Abschnitt (12) einen ersten Außendurchmesser definiert, der zweite Abschnitt einen zweiten Außendurchmesser definiert und der mittlere Abschnitt (14) einen dritten Außendurchmesser definiert, der kleiner ist als der erste und der zweite Außendurchmesser.

2. Vorrichtung nach Anspruch 1, wobei das Nitinol von 50 Gewichts-% bis 60 Gewichts-% Nickel und von 40 Gewichts-% bis 50 Gewichts-% Titan umfasst.

3. Vorrichtung nach einem der Ansprüche 1 und 2, wobei der mittlere Abschnitt (14) eine Länge von 5 bis 20 mm aufweist.

4. Vorrichtung nach einem der Ansprüche 1-3, wobei der erste Abschnitt (12) eine Länge von 20 bis 50 mm aufweist und der zweite Abschnitt (16) eine Länge von 5 bis 20 mm aufweist.

5. Vorrichtung nach einem der Ansprüche 1-3, wobei der mittlere Abschnitt (14) ein Nichtvorhandensein von Schraubengewindegängen aufweist.

6. Vorrichtung nach einem der Ansprüche 1-5, wobei die Knochenfixierungsvorrichtung (10) eine Zyklenermüdung von mindestens 400.000 Zyklen aufweist.

7. Vorrichtung nach einem der Ansprüche 1-6, wobei die Knocheneingriffselemente (22a/22b) Schraubengewindegänge (23a/23b) umfassen.

8. Vorrichtung nach einem der Ansprüche 1-7, wobei die Knochenfixierungsvorrichtung eine Schraube umfasst.

9. Vorrichtung nach einem der Ansprüche 1-8, weiter umfassend einen an den zweiten Abschnitt (16) angrenzenden Kopf, der einen Schlitz (35) zum Aufnehmen eines Werkzeugs umfasst.

10. Vorrichtung nach einem der Ansprüche 1-9, wobei der erste Abschnitt (12) einen zusätzlichen Schlitz zum Aufnehmen eines Werkzeugs umfasst.

11. Vorrichtung nach einem der Ansprüche 1-10, wobei sich die Knochenfixierungsvorrichtung (10) linear erstreckt.

12. Vorrichtung nach Anspruch 5, wobei die Strecke zwischen ungefähr 0,5 mm und ungefähr 3 mm beträgt.

13. Vorrichtung nach einem der Ansprüche 1-12, wobei der mittlere Abschnitt einen Elastizitätsmodul von 20 - 80 GPa aufweist.

## Revendications

1. Dispositif de fixation d'os (10) à des fins d'assujettissement flexible d'un tibia sur un péroné au niveau d'une syndesmose de la cheville comportant un corps monobloc en nitinol s'étendant dans le sens longitudinal ayant un axe s'étendant dans le sens longitudinal (17) et comportant : une première partie (12) comportant une surface extérieure ayant au moins un élément de mise en prise avec l'os (22a/22b) en mesure d'être assujetti sur le tibia ;
a. une seconde partie (16) comportant une surface extérieure ayant au moins un élément de mise en prise avec l'os en mesure d'être assujetti sur le péroné ; et
b. une partie centrale (14) s'étendant entre la première partie et la seconde partie et en mesure d'être positionnée entre le tibia et le péroné dans lequel la partie centrale (14) est configurée à des fins de déflexion en provenance de l'axe longitudinal (17) pour permettre au péroné de bouger par rapport au tibia dans une direction transversale par rapport à l'axe longitudinal selon une distance comparable au mouvement naturel du tibia par rapport au péroné au niveau de l'articulation syndesmose ; et
dans lequel la première partie (12) définit un premier diamètre extérieur, la seconde partie définit un second diamètre extérieur, et la partie centrale (14) définit un troisième diamètre extérieur inférieur aux premier et second diamètres extérieurs.

2. Dispositif selon la revendication 1, dans lequel le nitinol comporte de 50 % à 60 % de nickel, et de 40 % à 50 % de titane en poids.

3. Dispositif selon l'une quelconque des revendications précédentes 1 et 2, dans lequel la partie centrale (14) a une longueur de 5 à 20 mm.

4. Dispositif selon l'une quelconque des revendications 1 à 3, dans lequel la première partie (12) a une longueur de 20 à 50 mm et la seconde partie (16) a une longueur de 5 à 20 mm.

5. Dispositif selon l'une quelconque des revendications 1 à 3, dans lequel la partie centrale (14) présente une absence de filetages de vis.

6. Dispositif selon l'une quelconque des revendications 1 à 5, dans lequel le dispositif de fixation d'os (10) présente une fatigue cyclique d'au moins 400 000 cycles.

7. Dispositif selon l'une quelconque des revendications 1 à 6, dans lequel les éléments de mise en prise avec l'os (22a/22b) comportent des filetages de vis (23a/23b).

8. Dispositif selon l'une quelconque des revendications 1 à 7, dans lequel le dispositif de fixation d'os comporte une vis.

9. Dispositif selon l'une quelconque des revendications 1 à 8, comportant par ailleurs une tête adjacente à la seconde partie (16) comportant une fente (35) à des fins de réception d'un outil.

10. Dispositif selon l'une quelconque des revendications 1 à 9, dans lequel la première partie (12) comporte une fente supplémentaire à des fins de réception d'un outil.

11. Dispositif selon l'une quelconque des revendications 1 à 10, dans lequel le dispositif de fixation d'os (10) s'étend linéairement.

12. Dispositif selon la revendication 5, dans lequel la distance est entre environ 0,5 mm et environ 3 mm.

13. Dispositif selon l'une quelconque des revendications 1 à 12, dans lequel la partie centrale a un module d'élasticité de 20 à 80 GPa.
